# DEMANDE DE BREVET EUROPEEN

(11) **EP 1 228 746 A1**
(43) Date de publication de la demande: **07.08.2002**
(21) Numéro de dépôt: 02290213.4
(22) Date de dépôt: 30.01.2002
(51) Int. Cl.: A61K 7/00, B01J 13/00

(54) **Suspension de nanosphères de principe actif lipophile stabilisée des polymères hydrodispersibles**

(30) Priorité: 02.02.2001 FR 0101438
(71) Demandeur: L'OREAL, 75008 Paris (FR)
(72) Inventeur: Simmonnet, Jean-Thierry, 75011 Paris (FR)
(74) Mandataire: Casalonga, Axel

(57) **Abrégé**

La présente invention concerne une suspension colloïdale stable constituée essentiellement :
- d'une phase aqueuse continue,
- de nanosphères de principe actif lipophile ayant une taille moyenne de particules allant de 0,01 à 1 µm,
- d'au moins un agent tensioactif, et
- de particules colloïdales ayant une taille moyenne allant de 10 à 500 nm, d'au moins un polymère hydrodispersible,
ainsi que des compositions à application topique contenant une telle suspension colloïdale.

L'invention concerne également l'utilisation de polymères hydrodispersibles sous forme de particules colloïdales ayant une taille moyenne allant de 10 à 500 nm pour stabiliser des suspensions aqueuses de nanosphères de principe actif lipophile, ayant une taille moyenne de particules allant de 0,01 nm à 1 µm, contre la recristallisation du principe actif.

## Description

La présente invention concerne des suspensions aqueuses de nanosphères de principes actifs lipophiles, stabilisées par des particules de polymères hydrodispersibles, ainsi que des compositions à application topique les contenant.

Il existe de nombreux principes actifs cosmétiques qui, du fait de leur caractère fortement lipophile, sont insolubles dans la plupart des solvants cosmétiquement acceptables et sont par conséquent difficiles à incorporer dans des compositions cosmétiques.

Une approche intéressante pour résoudre ce problème d'insolubilité consiste à former avec ces molécules lipophiles des particules solides de très petite taille (inférieure au micron), appelées nanoparticules, que l'on peut ensuite utiliser sous forme de suspensions aqueuses colloïdales.

Parmi les nanoparticules, on distingue les nanocapsules et les nanosphères.
Les nanocapsules sont des particules présentant une structure coeur-enveloppe. La phase interne contenant le principe actif sous forme solubilisée, dispersée ou pure, est encapsulée dans une enveloppe continue, solide, insoluble dans le milieu, et qui est généralement de nature polymérique ou cireuse.
Les nanosphères sont des sphères pleines constituées du principe actif solide pur ou du principe actif incorporé dans une matrice cireuse ou polymérique.

Les nanosphères considérées dans la présente invention sont celles constituées du principe actif solide pur. Le principe actif y est sous forme amorphe. Néanmoins, au-delà d'une certaine concentration critique en principe actif, cette forme amorphe peut évoluer par recristallisation de l'actif en question.

D'une façon générale, l'instabilité des suspensions aqueuses de nanosphères de principe actif lipophile se traduit par l'agrégation des particules entre elles et/ou la recristallisation non contrôlée du principe actif, notamment sous l'effet de ce que l'on appelle "le murissement d'Ostwald" comme décrit par exemple dans l'article de KABALNOF *et al*. *J*. *Colloid and Interface Sci*., 118 (1987), pages 590 à 597. Cette évolution se traduit à terme par une sédimentation des particules voire une prise en masse de celles-ci conduisant naturellement à une moindre biodisponibilité du principe actif si celui-ci est dispersé dans un support cosmétique ou dermatologique.

Or, l'impossibilité de préparer des suspensions aqueuses stables avec des teneurs importantes en principe actif représente un inconvénient considérable pour le formulateur en cosmétique. En effet, l'addition du principe actif sous forme de suspensions diluées implique l'introduction indésirable d'une fraction importante de phase aqueuse susceptible de modifier les propriétés physico-chimiques de la composition cosmétique.
Il subsiste donc le besoin de disposer de suspensions aqueuses stables contenant des concentrations élevées de nanosphères de principe actif lipophile.

La demanderesse a donc cherché à formuler des principes actifs lipophiles, biologiquement actifs, sous forme de suspensions aqueuses stables de nanosphères ayant des dimensions inférieures au micron et à forte concentration en principe actif.

La demanderesse a découvert le fait surprenant qu'il était possible de stabiliser des suspensions aqueuses de nanosphères de principes actifs lipophiles sous forme amorphe en y ajoutant des particules colloïdales d'au moins un polymère hydrodispersible. L'ajout d'un polymère hydrodispersible, le plus souvent sous forme de particules colloïdales en suspension aqueuse, à une suspension aqueuse de nanosphères de principe actif permet en effet d'augmenter de manière significative la concentration critique au-delà de laquelle les nanosphères de principe actif en suspension présentent une tendance à la cristallisation non contrôlée.

La présente invention a par conséquent pour objet une suspension colloïdale stable constituée essentiellement :
- d'une phase aqueuse continue,
- de nanosphères de principe actif lipophile ayant une taille moyenne de particules allant de 0,01 à 1 µm (soit 10 nm à 1 µm),
- d'au moins un agent tensioactif, et
- de particules colloïdales ayant une taille moyenne allant de 10 à 500 nm, d'au moins un polymère hydrodispersible.
   Le polymère hydrodispersible est de préférence présent en une quantité suffisante pour stabiliser les nanosphères contre la recristallisation du principe actif.

L'invention a également pour objet une composition à application topique contenant, dans un milieu physiologiquement acceptable, une telle suspension colloïdale stable.
On entend ici par "application topique" une application externe sur les matières kératiniques, que sont notamment la peau, le cuir chevelu, les cils, les sourcils, les ongles et les muqueuses. La composition peut être en particulier une composition cosmétique ou dermatologique.
On entend par "milieu physiologiquement acceptable" un milieu compatible avec les tissus cutanés et susceptible d'être appliqué sur tout le corps humain, et notamment sur la peau, le cuir chevelu, les cils, les sourcils, les ongles et les muqueuses.

L'invention a en outre pour objet l'utilisation de polymères hydrodispersibles sous forme de particules colloïdales ayant une taille moyenne allant de 10 à 500 nm pour stabiliser des suspensions aqueuses de nanosphères de principe actif lipophile, ayant une taille moyenne de particules allant de 0,01 à 1 µm, contre la recristallisation du principe actif.

Les principes actifs lipophiles utilisés dans les suspensions aqueuses de la présente l'invention ont une solubilité dans l'eau à température ambiante (25°C) inférieure à 0,01 %, et une solubilité inférieure à 7,5 % dans les alcools de Guerbet tels que l'octyldodécanol, et dans les glycols, comme par exemple la glycérine, les polyéthylèneglycols ou l'isoprèneglycol.
Ils sont par ailleurs solides à température ambiante et présentent de préférence une température de fusion supérieure à 100 °C.

On peut citer à titre d'exemples de principes actifs lipophiles mis sous forme de nanosphères en suspensions aqueuses selon la présente invention ceux appartenant aux familles suivantes :
(1) Les stérols d'origine végétale ou animale tels que le cholestérol, l'ergostérol, le campestérol, le stigmastérol, le brassicastérol et le sitostérol, les dérivés partiellement hydrogénés de ces stérols (= stanols), ainsi que leurs esters.
(2) la déhydroépiandrostérone (DHEA) et ses précurseurs et dérivés chimiques et biologiques;
   la déhydroépiandrostérone est un stéroïde naturel, produit essentiellement par les glandes corticosurrénales, correspondant à la formule 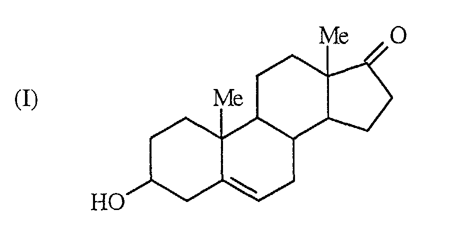
   Elle est connue pour ses propriétés anti-âge liées à sa capacité de promouvoir la kératinisation de l'épiderme (JP-07 196 467) et à lutter contre l'ostéoporose (US 5 824 671), ou encore dans le traitement des peaux sèches, en raison de son aptitude à augmenter la production endogène et la sécrétion de sébum et à renforcer l'effet barrière de la peau (US 4 496 556). Il a en outre été proposé d'utiliser le sulfate de DHEA contre l'alopécie (JP-60 142 908) et pour traiter différents signes du vieillissement tels que les rides, la perte d'éclat de la peau et le relâchement cutané (EP-0 723 775).
   La DHEA utilisable selon l'invention est par exemple disponible auprès des sociétés SIGMA et AKZO NOBEL.
   Par précurseur de la DHEA, on entend ses précurseurs biologiques immédiats ainsi que ses précurseurs chimiques. Des exemples de précurseurs biologiques sont le cholestérol, la prégnénolone, la 17α-hydroxyprégnénolone, le 5-androstènediol, le sulfate de 17α-hydroxyprégnénolone et le sulfate de 5-androstènediol. Des exemples de précurseurs chimiques sont les sapogénines telles que la diosgénine (spirost-5-èn-3-beta-ol), l'hécogénine, la smilagénine et la sarsapogénine, ainsi que des extraits naturels en contenant, en particulier le fénugrec et les extraits de Dioscorées telles que la racine d'igname sauvage ou *Wild Yam*.
   Par dérivés de la DHEA, on entend aussi bien ses dérivés métaboliques que ses dérivés chimiques. Comme dérivés métaboliques, on peut citer notamment la 7α-hydroxy-DHEA, la 7-céto-DHEA, le 5-androstène-3β-17β-diol (ou adiol), le sulfate de 5-androstène-3β-17β-diol et la 4-androstène-3,17-dione, sans que cette liste soit limitative. Comme dérivés chimiques, on peut citer notamment les sels, en particulier les sels hydrosolubles tels que le sulfate de DHEA, les esters tels que les esters d'acides hydroxycarboxyliques et de DHEA décrits dans US 5,736,537 ou les autres esters tels que le salicylate, l'acétate, le valérate et l'énanthate de DHEA ;
(3) les acides triterpène pentacycliques tels que l'acide ursolique et l'acide oléanolique. Ils sont présents dans les plantes telles que le romarin. Ils sont fréquemment utilisés dans les compositions pharmaceutiques pour leurs nombreuses propriétés thérapeutiques, et notamment pour leurs propriétés anti-inflammatoires, hépatoprotectrices, diurétiques, analgésiques, antimicrobiennes, inhibitrices de certaines activités enzymatiques et anti-tumorales. Dans le domaine cosmétique l'acide ursolique est décrit par exemple comme constituant d'une composition anti-transpirante (FR A 2 541 895) et comme inhibiteur de l'activité de la tyrosinase, enzyme clé de la synthèse mélanique (JP-58/57307).
(4) les hydroxystilbènes, qui sont des composés répondant à la formule générale : 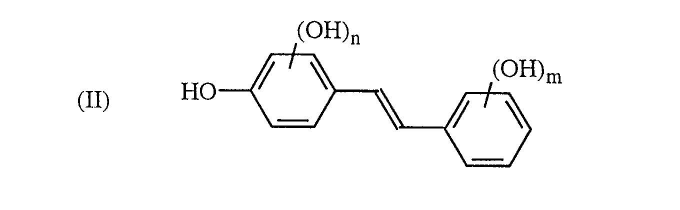 dans laquelle n est un nombre entier compris entre 1 et 4 inclusivement et m est un nombre entier compris entre 1 et 5 inclusivement. Cette formule englobe les composés *cis* et *trans*. Selon la présente invention, le terme hydroxystilbène recouvre également les dérivés hydroxyalkylés des composés de formule (II).
   Les hydroxystilbènes sont des composés que l'on retrouve à l'état naturel dans les végétaux de la classe des spermatophytes et en particulier dans la vigne. Dans le domaine cosmétique, les hydroxystilbènes sont utilisés, entre autres, comme agents dépigmentants (JP-87-192040) ou agents anti-âge (FR-2 777 186).
   Parmi les hydroxystilbènes, on peut citer les mono-, di-, tri-, tétra-, penta-hexa-, hepta-, octo- et nonahydroxystilbènes, ou encore leurs dérivés hydroxyalkylés.
   Selon l'invention, les hydroxystilbènes peuvent être utilisés seuls ou en mélanges de toute nature et peuvent être d'origine naturelle ou synthétique.
   Les hydroxystilbènes utilisables selon l'invention sont choisis parmi :
   le 4'-hydroxystilbène,
   le 2',4'-dihydroxystilbène,
   le 3',4'-dihydroxystilbène,
   le 4,4'-dihydroxystilbène,
   le 2',4',4-trihydroxystilbène,
   le 3',4',4-trihydroxystilbène,
   le 2,4,4'-trihydroxystilbène,
   le 3,4,4'-trihydroxystilbène,
   le 3,5,4'-trihydroxystilbène,
   le 2',3,4-trihydroxystilbène,
   le 2,3',4-trihydroxystilbène,
   le 2',2,4'-trihydroxystilbène,
   le 2,4,4',5'-tétrahydroxystilbène,
   le 2',3,4',5-tétrahydroxystilbène,
   le 2,2',4,4'-tétrahydroxystilbène,
   le 3,3',4',5-tétrahydroxystilbène,
   le 2,3',4,4'-tétrahydroxystilbène,
   le 3,3',4,4'-tétrahydroxystilbène,
   le 3,3',4',5,5'-pentahydroxystilbène,
   le 2,2',4,4',6-pentahydroxystilbène,
   le 2,3',4,4',6-pentahydroxystilbène,
   le 2.2',4,4',6,6'-hexahydroxystilbène.

   Parmi ces composés, le resvératrol (3,5,4'-trihydroxystilbène) est particulièrement préféré parce qu'il est naturellement présent dans la peau des grains de raisin et dans le vin. A cet égard, on peut consulter la revue de Soleas et collaborateurs, (Clinical Biochemistry, vol. 30, n° 2, pages 91 - 113, 1997) qui résume parfaitement l'état des connaissances concernant ce composé et les hydroxystilbènes.
(5) les isoflavonoïdes, sous-classe des flavonoïdes, qui sont formés d'un squelette 3-phénylchromane plus ou moins oxydé et pouvant porter différents substituants. Le terme isoflavonoïde regroupe plusieurs classes de composés parmi lesquels on peut citer les isoflavones, les isoflavanones, les roténoïdes, les ptérocarpans, les isoflavanes, les isoflavane-3-ènes, les 3-arylcoumarines, les 3-aryl-4-hydroxycoumarines, les coumestanes, les coumaronochromones, ou encore les 2-arylbenzofuranes. Une revue complète des isoflavonoïdes, de leurs sources et des méthodes d'analyse a été publiée dans « The Flavonoids », Harbone éditeur (1988), chapitre 5 intitulé « Isoflavonoids» par P.M. Dewick, pages 125 - 157.
   Les isoflavonoïdes utilisés selon l'invention ont une solubilité dans l'eau à température ambiante (25°C) inférieure à 0,01 % et peuvent être d'origine naturelle, c'est-à-dire extraits d'un élément d'origine naturelle, le plus souvent une plante, ou avoir été obtenus par synthèse chimique. On préfère les isoflavonoïdes d'origine naturelle.
   On peut citer à titre d'exemples d'isoflavonoïdes d'origine naturelle la génistine.
   Une sous-classe préférée des isoflavonoïdes est celle des isoflavones englobant aussi bien les formes aglycones (daidzéine, génistéine, glycitéine que les formes glycosylées (daidzine, génistine, glycitine).
   Des procédés de préparation d'isoflavones sont notamment décrits dans les brevets et demandes de brevet WO 95/10530, WO 95/10512, US 5 679 806, US 5 554 519, EP 812 837 et WO 97/26269.
   Les isoflavones sont connues en particulier en tant qu'agent anti-oxydants, pour leur propriétés anti-radicalaires et dépigmentantes, ainsi que pour leur capacité à inhiber l'activité des glandes sébacées (DE-44 32 947). Elles ont également été décrites en tant qu'agents capables de prévenir les signes de vieillissement de la peau (JP1-96106).
(6) les dérivés d'aminophénol de formule 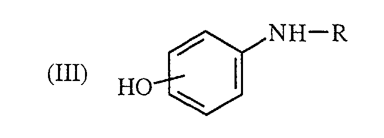
dans laquelle R est un radical correspondant à l'une des formules (i), (ii)
ou (iii) suivantes

(i) -CO-NR₁R₂

(ii) -CO-O-R₃

(iii) - SO₂-R₃

où
R₁ représente un atome d'hydrogène ou un radical alkyle en C₁₋₆, linéaire ou ramifié, saturé ou insaturé, éventuellement hydroxylé,
R₂ représente un atome d'hydrogène ou un radical choisi parmi les radicaux alkyle, saturés ou insaturés, linéaires, cycliques ou ramifiés, en C₁₂ à C₃₀, éventuellement hydroxylés, et
R₃ représente un radical choisi parmi les radicaux alkyle en C₁₂ à C₃₀, saturés ou insaturés, linéaires, ramifiés ou cycliques, y compris polycycliques condensés, et éventuellement hydroxylés.

On peut citer à titre d'exemples de principes actifs préférés selon la présente invention la déhydroépiandrostérone (DHEA), le sulfate de DHEA, la 7α-hydroxy-DHEA, la 7-céto-DHEA, la prednisolone, la prednisone, la progestérone, la prégnénolone, la testostérone, la diosgénine, l'hécogénine, l'acide ursolique, l'acide oléanolique, le resvératrol (= 3,5,4'-trihydroxystilbène) et le N-cholestéryloxycarbonyl-4-aminophénol, les isoflavonoïdes dont la solubilité dans l'eau à température ambiante (25 °C) est inférieure à 0,01 %.

Les suspensions colloïdales de la présente invention contiennent, en plus de la phase aqueuse et des nanosphères de principe actif lipophile un ou plusieurs agents tensioactifs choisis parmi les agents tensioactifs non ioniques, anioniques, cationiques ou zwitterioniques, ou des mélanges de ceux-ci. Ces agents tensioactifs sont introduits pendant le procédé de préparation des suspensions aqueuses de nanosphères. Le ou les agents tensioactifs, selon leur caractère hydrophile ou lipophile, sont alors dissous dans la phase aqueuse et/ou organique. Dans la suspension aqueuse de nanosphères finale, les agents tensioactifs peuvent se retrouver dissous dans la phase continue aqueuse, adsorbés à la surface des nanosphères ou bien incorporés dans les nanosphères de principe actif lipophile.

La concentration globale du ou des agents tensioactifs utilisé(s) va de préférence de 0,02 à 25 %, et mieux de 0,05 à 10 %, rapportée au poids total de la suspension aqueuse de nanosphères finale.

On peut citer à titre d'exemples d'agents tensioactifs préférés utilisables dans les suspensions aqueuses de nanosphères selon la présente invention ceux qui font partie des familles suivantes :
(a) les phospholipides naturels ou de synthèse, hydrogénés ou non, enrichis ou non en phosphatidylcholine. On peut citer à titre d'exemple la lécithine de soja enrichie avec 45 % en poids de phosphatidylcholine (commercialisée sous la dénomination Emulmetik® 900 par la société LUCAS MEYER) ou une lécithine de soja hydrogénée (commercialisée sous la dénomination Lécinol® S10 par la société NIKKOL ou sous la dénomination Emulmetik® 950 par la société LUCAS MEYER) ;
(b) les stérols polyéthoxylés tels que le cholestérol et le phytostérol polyéthoxylés par 5 à 100 motifs d'oxyde d'éthylène (OE) ;
(c) les agents tensio-actifs choisis parmi les esters gras de glycérol, les esters gras de sorbitane, les esters gras de sorbitane polyéthoxylés, les alcools gras polyéthoxylés et les acides gras polyéthoxylés, les chaînes grasses de ces molécules étant des chaînes saturées, linéaires ou ramifiées en C₁₂₋₃₀; on peut citer à titre d'exemples l'alcool béhénylique polyéthoxylé à 30 OE, l'acide stéarique polyéthoxylé à 40 OE ou le laurate de sorbitane polyéthoxylé à 20 OE ;
(d) le poly(alcool vinylique), la polyvinylpyrrolidone et leurs copolymères ;
(e) les polysiloxanes polyéthoxylés, éventuellement polypropoxylés (dénomination CTFA : diméthicone copolyols), comme par exemple ceux décrits dans les brevets US-A-5 364 633 et US-A-5 411 744. Ces agents tensio-actifs siliconés correspondent en particulier à la formule 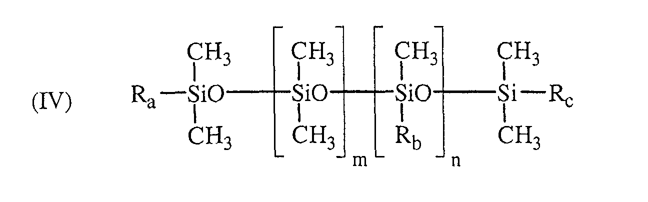 dans laquelle Rₐ, R_{b} et R_{c} représentent chacun indépendamment un radical alkyle en C₁₋₆ ou un radical -(CH₂)ₓ-(O-CH₂CH₂)_{y}-(OCH₂CH₂CH₂)_{z}-OR_{d} où R_{d} représente un atome d'hydrogène ou un radical alkyle ou acyle, au moins un des radicaux Rₐ, R_{b} et R_{c} n'étant pas un radical alkyle en C₁₋₆,
   m est un nombre entier allant de 0 à 200,
   n est un nombre entier allant de 0 à 50, la somme m + n étant différente de zéro,
   x est un nombre entier allant de 1 à 6,
   y est un nombre entier allant de 1 à 30, et
   z est un nombre entier allant de 0 à 5.

Selon un mode de réalisation préféré de l'invention, les radicaux alkyle Rₐ, R_{b}, R_{c} ou R_{d} représentent un groupe méthyle, x est un nombre entier allant de 2 à 6 et y un nombre entier allant de 4 à 30. On peut citer à titre d'exemples d'agents tensioactifs siliconés de formule (IV) les composés de formule (IVa) 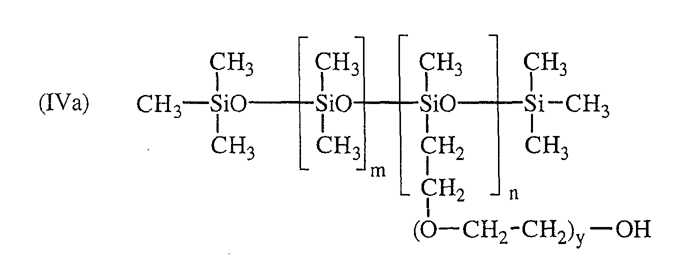 dans laquelle m est un nombre entier allant de 20 à 105, n est un nombre entier allant de 2 à 10 et y est un nombre entier allant de 10 à 20,
ou les composés de formule (IVb) 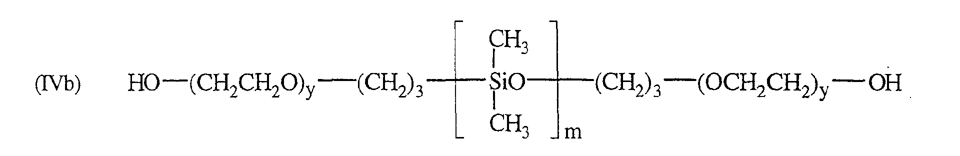 dans laquelle m et y sont des nombres entiers allant de 10 à 20.

On peut utiliser notamment comme agents tensioactifs siliconés ceux commercialisés par la société DOW CORNING sous les dénominations DC 5329 (composé de formule (IVa) où m = 22, n = 2, y = 12), DC 7439-146 (composé de formule (IVa) où m = 103, n = 10, y = 12), DC 2-5695 (composé de formule (IVa) où m = 27, n = 3, y = 12) et Q4-3667 (composé de formule (IIb) où m = 15 et y = 13).
(f) les copolymères diséquencés d'oxyde d'éthylène et d'oxyde de propylène ;
(g) les copolymères diséquencés de styrène et d'oxyde d'éthylène tels que par exemple les produits commercialisés par la société GOLDSCHMIDT sous les dénominations SE0418 (PS400/OE1800), SE0720 (PS700/OE2000), SE1010 (PS1000/OE1000), SE1030 (PS1000/OE3000), ou encore les dérivés sulfatés anioniques de ces copolymères tels que le SE1030A commercialisé par la société Goldschmidt ;
(h) les esters d'acides gras et de sucres et les éthers d'alcools gras et de sucres et notamment les esters d'acides gras en C₈₋₂₂ et de saccharose, de maltose, de glucose ou de fructose, ou les esters d'acides gras en C₁₄₋₂₂ et de méthylglucose ;
(i) les alcényl(C₁₂₋₃₀)succinates choisis parmi les alcénylsuccinates polyalcoxylés, les alcénylsuccinates de glycose polyalcoxylés et les alcénylsuccinates de méthylglucose polyalcoxylés, comme par exemple l'hexadécénylsuccinate de PEG (18 ou 45 OE) et le dihexadécénylsuccinate PEG (18 OE) ;
(j) les acétylènediols polyéthoxylés tels que le 2,4,7,9-tétraméthyl-5-décyne-4,7-diol éthoxylé (1,3 OE) commercialisé par la société Air Product Chemical sous la dénomination Surfinol® 402 ;
(k) les alkyl(C₁₂₋₃₀)éthersulfates de sodium tels que le lauryléthersulfate de sodium (en moyenne 2,2 OE), et les alkyl(C₁₂₋₃₀)sulfates de sodium tels que le laurylsulfate de sodium, le tridécylsulfate de sodium et le cétylstéarylsulfate (50/50) de sodium ;
(l) les sels d'ammonium quaternaire ayant des propriétés tensioactives correspondant à la formule suivante : 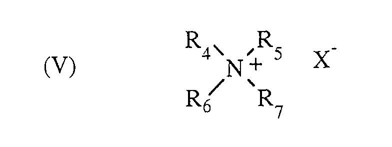
dans laquelle les radicaux R₄, R₅, R₆ et R₇ représentent chacun indépendamment un groupe aliphatique, linéaire ou ramifié ou un groupe aromatique tel qu'un noyau aryle ou alkylaryle, comportant de 1 à 30 atomes de carbone ; les groupes aliphatiques peuvent comporter des hétéroatomes tels qu'un atome d'oxygène, d'azote, de soufre ou d'halogène, et sont choisis par exemple parmi les radicaux alkyle, alcoxy, polyoxy(alkylène en C₂₋₆), alkylamide, (alkyle en C₁₂₋₂₂)-amido-(alkyle en C₂₋₆), (alkyle en C₁₂₋₂₂)-acétate
ou hydroxyalkyle, comportant de 1 à environ 30 atomes de carbone ;
X représente un anion choisi parmi les ions halogénures, phosphates, acétates, lactates, alkyl(C₂₋₆)-sulfates, alkyl(C₂₋₆)- ou alkyl(C₂₋₆)-arylsulfonates.

Les principes actifs lipophiles peuvent être mis sous forme de suspensions aqueuses de nanosphères ayant une taille moyenne allant de 0,01 à 1 µm notamment selon deux procédés connus.

Le premier procédé, appelé "nanoprécipitation par basculement de solvant" (en anglais *solvent nanoprecipitation*) est décrit par exemple dans la demande de brevet EP-A-0 274 961.

Appliqué à la préparation des suspensions aqueuses de nanosphères de la présente invention, ce procédé de nanoprécipitation par basculement de solvant consiste
- à dissoudre le principe actif lipophile en une concentration allant de 0,1 à 30 % en poids dans un solvant organique plus volatil que l'eau et miscible avec celle-ci,
- à introduire, sous agitation modérée, la solution organique du principe actif dans une phase aqueuse, l'une au moins de ces deux phases contenant au moins un agent tensioactif dissous en une concentration supérieure ou égale à sa concentration micellaire critique, puis
- à évaporer le solvant organique plus volatil que l'eau, ainsi qu'éventuellement une partie de l'eau.

Dans ce procédé, grâce à la présence d'au moins un agent tensioactif, les nanosphères se forment spontanément par précipitation du principe actif lors du mélange de la solution organique du principe actif et de la phase aqueuse. Le volume de phase aqueuse doit être suffisant pour l'obtention d'une précipitation satisfaisante du principe actif lipophile. Dans la pratique il n'est jamais inférieur à celui de la solution organique.
Le rapport en poids phase aqueuse/phase organique est au moins égal à 1 et va de préférence de 1 à 20.

La taille des nanosphères dépend notamment de la nature du solvant, de la concentration du principe actif dans celui-ci, du rapport phase organique/phase aqueuse, ainsi que de la nature et de la quantité de l'agent tensio-actif.
On peut citer comme solvants plus volatils que l'eau et miscibles avec celle-ci les cétones telles que l'acétone, les alcools en C₁₋₆ tels que le méthanol ou l'isopropanol, le tétrahydrofurane, ainsi que des mélanges de ces solvants.

Le deuxième type de procédé de préparation des suspensions aqueuses de nanosphères de la présente invention se distingue du premier procédé décrit ci-dessus principalement par la non-miscibilité de la phase organique avec la phase aqueuse. Le mélange de la solution organique du principe actif lipophile et de la phase aqueuse donnera donc lieu à une émulsion huile-dans-eau.

Ce procédé dit par émulsification consiste en particulier
- à dissoudre le principe actif lipophile en une concentration allant de 0,1 à 30 % en poids dans un solvant organique plus volatil que l'eau et non miscible avec celle-ci,
- à émulsifier la solution organique du principe actif avec une phase aqueuse, l'une au moins de ces deux phases contenant au moins un agent tensio-actif en une concentration supérieure ou égale à sa concentration micellaire critique, puis
- à évaporer rapidement le solvant organique plus volatil que l'eau, et éventuellement une partie de l'eau.

Les solvants organiques plus volatils que l'eau et non miscibles avec celle-ci sont choisis par exemple parmi les hydrocarbures halogénés comme le dichlorométhane, ou les hydrocarbures cycliques tels que le cyclohexane et le toluène.
Le rapport pondéral phase aqueuse/phase organique lors de l'étape d'émulsification est compris entre 1,5 et 99.
Contrairement à ce qui se passe pour le premier type de procédé, les nanosphères ne se forment pas spontanément mais il est généralement nécessaire d'affiner la préémulsion obtenue en la faisant passer une ou plusieurs fois dans un homogénéisateur haute pression (10 à 120 MPa) ou en l'exposant à des ultrasons. La taille des nanosphères obtenues dépendra directement de l'efficacité de cette étape d'émulsification poussée.

Les suspensions aqueuses de nanosphères obtenues selon l'un des deux types de procédés décrits ci-dessus peuvent être concentrées par élimination d'une certaine quantité de la phase aqueuse. Cette élimination peut se faire par exemple par évaporation sous vide ou par ultrafiltration.

On recherchera généralement des concentrations importantes en principe actif pour lesquelles la stabilité des nanosphères en suspension reste satisfaisante, autrement dit on ajustera de préférence la teneur en principe actif des suspensions aqueuses de nanosphères à une valeur allant de 0,2 à 50 % en poids, de préférence de 1 à 20 % en poids en poids, par rapport au poids total de la suspension aqueuse de nanosphères.

La faible taille moyenne des nanosphères est essentielle pour la bonne biodisponibilité des principes actifs lipophiles et l'on préfère en particulier des nanosphères ayant une taille moyenne allant de 50 à 500 nm.

L'addition selon l'invention d'un ou de plusieurs polymères hydrodispersibles aux suspensions aqueuses de nanosphères se fait après la préparation de celles-ci, c'est-à-dire après évaporation de la phase organique volatile.
Lorsque les suspensions aqueuses de nanosphères sont concentrées par évaporation ou ultrafiltration d'une fraction de la phase aqueuse, l'addition des particules de polymère hydrodispersible précède de préférence l'étape de concentration. Le polymère hydrodispersible peut cependant également être ajouté après concentration de la suspension aqueuse.

Il ressort de ce qui précède que les suspensions aqueuses de la présente invention contiennent deux types de particules nanométriques, à savoir des nanosphères de principe actif lipophile et des nanoparticules de polymères hydrodispersibles. Cette structure est par conséquent différente de celle des nanosphères composites décrites dans l'art antérieur où le principe actif se trouve incorporé dans une matrice polymérique. De telles nanosphères composites sont décrites par exemple dans l'article de SEIJO *et al*., *International Journal of Pharmaceutics*, 62 (1990), pages 1 - 7, et dans l'article de PAUL *et al*., *International Journal of Pharmaceutics*, 159, (1997), pages 223 - 232.

On entend par polymères hydrodispersibles selon la présente invention, des polymères insolubles dans l'eau qui, lorsqu'on les disperse, sous une agitation modérée à vive, dans de l'eau à une température comprise entre 10 et 90 °C forment spontanément des particules colloïdales ayant une taille moyenne allant de 10 à 500 nm.
On préfère en particulier utiliser selon la présente invention des particules colloïdales de polymère hydrodispersible ayant une taille moyenne allant de 20 à 400 nm.

Les polymères hydrodispersibles utilisés de préférence selon la présente invention sont des polymères, synthétiques ou d'origine naturelle, portant des charges anioniques.

Comme polymères anioniques hydrodispersibles d'origine naturelle susceptibles d'être utilisés selon la présente invention, on peut citer par exemple les dérivés anioniques de cellulose, et en particulier les esters et éthers anioniques de cellulose tels que l'acétophtalate de cellulose, l'acétosuccinate de cellulose, le propionosuccinate de cellulose, le butyrosuccinate de cellulose, l'acétopropionosuccinate de cellulose, l'acétotrimellitate de cellulose, l'acétopropionotrimellitate de cellulose, l'acétobutyrotrimellitate de cellulose et la carboxyméthylcellulose.

D'autres polymères hydrodispersibles anioniques d'origine naturelle utilisables sont la résine shellac, la gomme de sandaraque et les dammars.
La résine shellac est une sécrétion animale, composée principalement de résine et de cire et est soluble dans certains solvants organiques. Elle doit être sous-neutralisée pour ne pas devenir soluble dans l'eau.
La gomme de sandaraque est une résine extraite de l'écorce d'arbres tels que le *thuya articulata* ou le *callitris verrucosa*. Elle est composée principalement d'acides tels que l'acide pimarique, l'acide callitrolique et l'acide sandaricinique. Elle est insoluble dans l'eau mais peut être solubilisée dans des solvants organiques tels que l'éthanol, l'acétone ou l'éther.

Les dammars sont des résines provenant d'arbres des genres Damara
ou Shoréa et contiennent généralement 62,5 % de résines (40 % de solubles et 22,5 % d'insolubles dans l'alcool) et 23 % d'acides.

Les polymères hydrodispersibles anioniques utilisés dans la présente invention sont de préférence des polymères anioniques synthétiques et en particulier des polymères synthétiques choisis parmi les polyesters, poly(ester amide), polyuréthannes et copolymères vinyliques portant tous des fonctions acide carboxylique et/ou acide sulfonique.

Les polyesters anioniques sont obtenus par polycondensation de diacides carboxyliques aliphatiques, cycloaliphatiques et/ou aromatiques et de diols ou polyols aliphatiques, cycloaliphatiques et/ou aromatiques, un certain nombre de ces diacides et diols portant en outre une fonction acide carboxylique ou acide sulfonique libre ou sous forme de sel.
Comme diacides carboxyliques, on peut citer l'acide succinique, l'acide glutarique, l'acide adipique, l'acide pimélique, l'acide subérique, l'acide sébacique, l'acide téréphtalique, l'acide isophtalique ou l'anhydride de celui-ci.
Comme diols aliphatiques, on peut citer l'éthylèneglycol, le diéthylèneglycol. le triéthylèneglycol et le tétraéthylèneglycol, le di(hydroxyméthyl)-cyclohexane, le diméthylolpropane ou le 4,4'-(1-méthylpropylidène)-bis-phénol.
Les monomères polyols sont par exemple le glycérol, le pentaérytritol, le sorbitol.
Les comonomères permettant d'introduire des groupements anioniques sont par exemple l'acide diméthylolpropionique, l'acide trimellitique ou l'anhydride mellitique, ou un composé diol ou diacide carboxylique portant en plus un groupe SO₃M où M représente un atome d'hydrogène ou un ion d'un métal alcalin, tel que le 1,5-dihydroxypentane-3-sulfonate de sodium ou le 1,3-dicarboxybenzène-5-sulfonate de sodium.

Les poly(ester amide) anioniques utilisables dans la présente l'invention ont une structure similaire à celle des polyesters décrits ci-dessus mais contiennent en outre des motifs dérivés d'une diamine telle que l'hexaméthylènediamine, la méta- ou para-phénylènediamine, ou d'un aminoalcool tel que la méthanolamine.

Selon un mode de réalisation préféré de l'invention, le polymère anionique hydrodispersible est choisi parmi les polyesters aromatiques, cycloaliphatiques et/ou aliphatiques portant des fonctions acide sulfonique, c'est-à-dire des copolyesters comportant au moins des motifs dérivés d'acide isophtalique, d'acide sulfo-aryldicarboxylique et de diéthylèneglycol. Parmi ceux-ci, on peut citer tout particulièrement les polyesters comprenant des motifs dérivés d'acide isophtalique, d'acide sulfo-isophtalique, de diéthylèneglycol et de 1,4-di(hydroxyméthyl)cyclohexane, tels que ceux commercialisés sous les dénominations AQ®29, AQ®38, AQ®48 ULTRA, AQ®55S, AQ®1350, AQ®1045, AQ®1950 et AQ®14000 par la société EASTMAN CHEMICAL.

Ces polyesters peuvent aussi contenir, en plus de motifs dérivés d'acide isophtalique et d'acide sulfoisophtalique, des motifs dérivés d'éthylèneglycol, de triéthylèneglycol et/ou de tétraéthylèneglycol et d'acide téréphtalique comme ceux commercialisés sous les dénominations POLYCARE® PS 20, POLYCARE® PS30 et POLYCARE® PS 32 par la société RHONE POULENC.

La proportion de motifs dérivés d'acide sulfoisophtalique va de préférence de 2 à 20 % en poids.

Les polyuréthannes utilisables en tant que polymères anioniques hydrodispersibles sont par exemple des copolymères polyuréthanne-poly(acide acrylique) ou les copolymères polyuréthanne-polyester ou poly(ester uréthanne) anioniques.

Les copolymères vinyliques utilisables en tant que polymères hydrodispersibles anioniques englobent notamment des polymères filmogènes utilisés couramment pour la préparation de compositions cosmétiques parmi lesquels on peut citer :
(i) les copolymères acétate de vinyle/acide crotonique polyéthoxylés tels que celui commercialisé sous la dénomination ARISTOFLEX® A par la société HOECHST ;
(ii) les copolymères acétate de vinyle/acide crotonique tels que celui commercialisé sous la dénomination LUVISET® CA66 par la société BASF ;
(iii) les terpolymères acétate de vinyle/acide crotonique/néodécanoate de vinyle tels que celui commercialisé sous la dénomination RÉSINE 28-29-30, par la société NATIONAL STARCH ;
(iv) les copolymères N-octylacrylamide/méthacrylate de méthyle/méthacrylate d'hydroxypropyle/acide acrylique/méthacrylate de tert-butylaminoéthyle tels que celui commercialisé sous la dénomination de AMPHOMER® par la société NATIONAL STARCH.
(v) les copolymères alternés méthylvinyléther/anhydride maléique monoestérifiés par le butanol, tels que celui commercialisé sous la dénomination GANTREZ® ES 425 par la société GAF ;
(vi) les terpolymères acide acrylique/acrylate d'éthyle/N-tert-butylacrylamide tels que celui commercialisé sous la dénomination ULTRAHOLD® 8 par la société BASF ;
(vii) les polymères répondant à la formule générale suivante 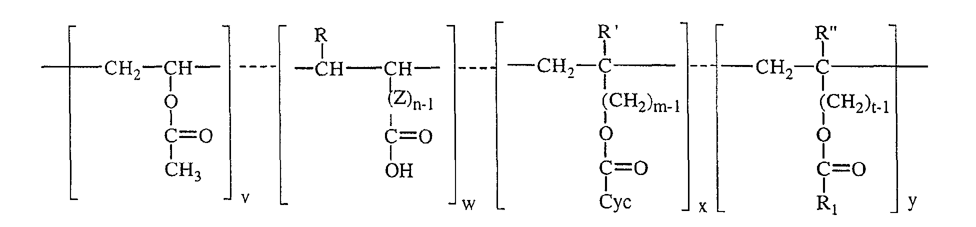
dans laquelle
R, R' et R", identiques ou différents, représentent un atome d'hydrogène ou un radical méthyle,
m, n et t valent 1 ou 2,
R₁ représente un radical alkyle en C₂₋₂₁, linéaire ou ramifié, saturé ou insaturé,
Z représente un radical divalent choisi parmi les résidus :

-CH₂-, -CH₂-O-CH₂- et CH₂-O-(CH₂)₂-,

*Cyc* représente un radical choisi parmi :
(a) un radical de formule 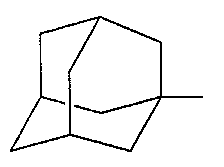
(b) un radical de formule 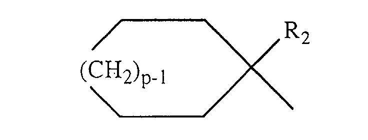 dans laquelle R₂ représente un atome d'hydrogène ou un radical méthyle, et p vaut 1 ou 2,
(c) un radical de formule 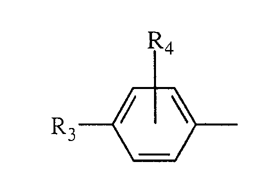 dans laquelle R₃ représente un atome d'hydrogène, un radical méthyle, éthyle, tert-butyle, éthoxy, butoxy ou dodécyloxy et R₄ représente un atome d'hydrogène, un radical alkyle en C₁₋₄ ou un radical alcoxy en C₁₋₄, et
(d) un radical de formule 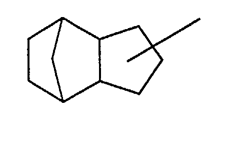 v est choisi de manière à ce que les motifs correspondants représentent de 10 à 91 % en poids, de préférence de 36 à 84 % en poids du polymère total,
   w est choisi de manière à ce que les motifs correspondants représentent de 3 à 20 % en poids, de préférence de 6 à 12 % en poids du polymère total,
   x est choisi de manière à ce que les motifs correspondants représentent de 4 à 60 % en poids, de préférence de 6 à 40 % en poids du polymère total et
   y est choisi de manière à ce que les motifs correspondants représentent de 0 à 40 % en poids, de préférence de 4 à 30 % en poids du polymère total la somme de v + w + x + y étant égale à 100 %.

Parmi ces polymères, on peut citer notamment le copolymère acétate de vinyle/tert-butyl-4-benzoate de vinyle/acide crotonique (65/25/10) neutralisé à 50 - 60 % par la lysine et le copolymère acétate de vinyle/acide crotonique/tert-butyl-4-benzoate de vinyle (65/10/25) neutralisé à 60 % par la lysine.

La masse molaire moyenne en poids des polymères anioniques hydrodispersibles utilisés dans la présente invention pour la stabilisation de suspensions de nanosphères de principe lipophile va généralement de 1000 à 5 000 000, de préférence de 5000 à 500 000.

Comme indiqué ci-dessus, les polymères hydrodispersibles anioniques décrits ci-dessus doivent être insolubles dans l'eau. Or, la présence des charges anioniques confère aux polymères un caractère polaire et favorise leur dissolution dans l'eau. Il est par conséquent indispensable de limiter le taux de charge des polymères.
Ce taux de charge limite supérieur qu'il convient de ne pas dépasser pour que le polymère reste insoluble dépend
- de la nature chimique du polymère, c'est-à-dire du caractère hydrophobe des motifs le composant,
- de la masse molaire du polymère, un polymère de faible masse molaire étant généralement plus soluble dans l'eau qu'un polymère de forte masse, ou encore
- de la nature de l'agent de neutralisation des fonctions acides.

Il est possible de modifier ce taux de charge en jouant sur la teneur en comonomères introduisant des fonctions acide carboxylique ou acide sulfonique ou sur le taux de neutralisation des groupements acide faible (groupements acide carboxylique).

La neutralisation partielle (ou sous-neutralisation) des fonctions acide faible, peut se faire par addition d'un agent monobasique non volatil, tel qu'une base minérale comme la soude ou la potasse, ou un aminoalcool pris dans le groupe constitué par l'amino-2-méthyl-2-propanol-1 (AMP), la triéthanolamine (TEA), la triisopropanolamine (TIPA), la monoéthanolamine, la diéthanolamine, la tri[(hydroxy-2)propyl-1]-amine, l'amino-2-méthyl-2-propanediol-1,3 (AMPD) et l'amino-2-hydroxyméthyl-2-propanediol-1,3.

On peut ainsi neutraliser d'environ 20 à 80 % des groupements ionisables pour stabiliser la dispersion aqueuse du polymère sans solubiliser le polymère.

Dans la présente demande, on entend par "quantité suffisante pour stabiliser les nanosphères contre la recristallisation du principe actif" une quantité de polymère hydrodispersible permettant d'obtenir des suspensions aqueuses ne présentant aucun signe d'évolution visible au microscope (polarisation croisée, contraste de phase) à un grossissement adapté à la taille des particules, et en particulier aucun signe de recristallisation du principe actif après au moins 7 jours de conservation à une température comprise entre 4 °C et 45 °C.

La quantité de polymère hydrodispersible nécessaire pour obtenir une stabilisation satisfaisante des suspensions aqueuses de nanosphères de principe actif lipophile dépend de nombreux paramètres tels que le taux de charge et la nature chimique du polymère hydrodispersible, la nature chimique et la concentration de principe actif ou encore la nature et la concentration de l'agent tensioactif utilisé.

La demanderesse a constaté qu'un rapport pondéral polymère hydrodispersible/principe actif lipophile allant de 1/100 à 1/1 donne généralement des résultats satisfaisants, c'est-à-dire des suspensions aqueuses ne présentant pas de signes de cristallisation après 7 jours et même après 2 mois et plus de conservation à une température comprise entre 4 et 45 °C.

Ce rapport pondéral polymère hydrodispersible/principe actif lipophile va plus préférentiellement de 1/50 à 1/2.

La présente invention a également pour objet des compositions à application topique contenant une suspension aqueuse de nanosphères de principe actif lipophile stabilisée par des polymères hydrodispersibles.
Ces compositions à application topique contiennent de 0,1 à 40 % en poids, et de préférence de 1 à 30 % en poids de suspension aqueuse de nanoparticules de principe actif, dans un milieu physiologiquement acceptable.
Ces compositions peuvent se présenter par exemple sous forme de lotions, de gels, de suspensions, d'émulsions telles que E/H, H/E, d'émulsions multiples E/H/E, H/E/H, ou de nanoémulsions.
Elles peuvent contenir des additifs et adjuvants utilisés habituellement dans le domaine cosmétique tels que des agents antioxydants, des huiles essentielles, des agents hydratants, des vitamines, des acides gras essentiels, des sphingolipides, des agents autobronzants, des agents anti-radicalaires, des filtres solaires, des parfums, des conservateurs, des colorants, des agents anti-mousse, des agents séquestrants, des agents d'ajustement du pH, des agents épaississants hydrophiles tels que les polysaccharides (gomme de xanthane), les Carbomers (polymères carboxyvinyliques), l'acide polyacrylamidométhylpropanesulfonique partiellement neutralisé et hautement réticulé.
Bien entendu, l'homme du métier veillera à choisir les éventuels composés complémentaires de manière à ce que les propriétés avantageuses de la composition selon l'invention, et notamment la stabilité des nanosphères, ne soient pas, ou pratiquement pas, altérées par l'adjonction envisagée.

La composition selon l'invention peut être utilisée dans de nombreuses applications cosmétiques ou dermatologiques où la présence d'actifs lipophiles est utile, notamment pour le traitement, le soin et/ou le maquillage de la peau du visage et/ou du corps, des muqueuses (lèvres), du cuir chevelu et/ou des fibres kératiniques (cheveux ou cils).

Ainsi, la composition de l'invention peut être utilisée comme produit de soin et/ou d'hygiène ou comme produit solaire pour le visage, les mains ou le corps. Elle peut constituer aussi un produit pour le maquillage des fibres kératiniques, de la peau, des lèvres et/ou des ongles.

La composition selon l'invention peut également être utilisée comme produit capillaire rincé ou non-rincé, notamment pour le lavage, le soin, le conditionnement, le maintien de la coiffure ou la mise en forme des fibres kératiniques telles que les cheveux.

Ainsi, la présente invention a également pour objet l'utilisation cosmétique de la composition selon l'invention, pour le traitement, le soin et/ou le maquillage de la peau du visage et/ou du corps, des muqueuses (lèvres), du cuir chevelu et/ou des fibres kératiniques.

Un autre objet de l'invention est un procédé de traitement cosmétique des matières kératiniques humaines comme la peau, y compris le cuir chevelu, les cheveux, les cils, les sourcils, les ongles ou les muqueuses notamment les lèvres, caractérisé par le fait que l'on applique sur les matières kératiniques, une composition cosmétique telle que définie ci-dessus, selon la technique d'utilisation habituelle de cette composition. Par exemple, application de crèmes, de gels, de sérums, de lotions, de laits sur la peau, le cuir chevelu, et/ou les muqueuses. Le type de traitement est fonction du ou des actifs présents dans la composition.

La présente invention sera illustrée à l'aide des exemples suivants.

### Exemple 1

### Stabilité de suspensions aqueuses de N-cholestéryloxycarbonyl-4-aminophénol

On prépare selon le procédé de nanoprécipitation par basculement de solvant décrit ci-dessus, une suspension aqueuse de nanoparticules de N-cholestéryloxycarbonyl-4-aminophénol stabilisée par un polymère hydrodispersible ainsi qu'une suspension comparative exempte de polymère hydrodispersible.

Ces suspensions sont conservées pendant 2 mois à une température de 45 °C, période au bout de laquelle on évalue le degré de cristallisation du principe actif par microscopie en polarisation et par microscopie en contraste de phase. La composition des suspensions et les résultats obtenus sont rassemblés dans le tableau ci-dessous.

| | Suspension A (selon l'invention) | Suspension comparative |
|---|---|---|
| N-cholestéryloxycarbonyl-4-aminophénol | 3 % en poids | 3% en poids |
| lécithine de soja | 0,5 % en poids | 0,5 % en poids |
| suspension aqueuse de AQ®38S^{*)} à 6 % en poids | 20 % en poids | - |
| eau distillée | q.s.p.100 % en poids | q.s.p.100 % en poids |
| résultat de l'examen microscopique | absence de cristallisation après 2 mois de conservation à 45 °C | cristallisation massive après 2 mois de conservation à 45 °C |

| | | |
|---|---|---|
| *) copolyester d'acide isophtalique, d'acide sulfoisophtalique, de diméthylolcyclohexane et de diéthylèneglycol commercialisé par la société EASTMAN CHEMICAL | | |

Ces résultats montrent que l'addition, à une suspension aqueuse de nanoparticules de N-cholestéryloxycarbonyl-4-aminophénol, de seulement 1,2 % en poids d'un polymère hydrodispersible selon la présente invention permet de stabiliser parfaitement la suspension pendant au moins 2 mois.

### Exemple 2

On prépare selon le procédé par émulsification décrit ci-dessus, une suspension aqueuse contenant 5 % en poids de nanoparticules de N-cholestéryloxycarbonyl-4-aminophénol, 3 % en poids de lécithine de soja et 1,2 % en poids de polymère hydrodispersible selon l'invention (AQ®38S). Après 2 mois de conservation de la suspension aqueuse à une température de 45 °C, l'examen par microscopie en polarisation croisée et par microscopie en contraste de phase, révèle l'absence de particules de principe actif cristallisées. Les nanosphères de N-cholestéryloxycarbonyl-4-aminophénol de la suspension ont une taille moyenne, mesurée par granulométrie laser (BI90 PLUS de BROOKHAVEN (méthode QLS)), de 131 nm, identique à celle mesurée pour la suspension avant conservation.

### Exemple 3

On prépare selon le procédé par émulsification décrit ci-dessus, une suspension aqueuse contenant 9,3 % en poids de nanoparticules de N-cholestéryloxycarbonyl-4-aminophénol, 4,8 % en poids de lécithine de soja et 1,2 % en poids de polymère hydrodispersible selon l'invention (AQ®38S). Après 2 mois de conservation de la suspension aqueuse à une température de 45 °C, l'examen par microscopie en polarisation croisée et par microscopie en contraste de phase, révèle l'absence de particules de principe actif cristallisées. Les nanosphères de N-cholestéryloxycarbonyl-4-aminophénol de la suspension ont une taille moyenne, mesurée par granulométrie laser (BI90 PLUS de BROOKHAVEN (méthode QLS)), de 112 nm, identique à celle mesurée pour la suspension avant conservation.

### Exemple 4

On prépare selon le procédé de nanoprécipitation par basculement de solvant décrit ci-dessus une suspension aqueuse contenant 1,5 % en poids de nanoparticules d'acide ursolique ayant une taille moyenne, mesurée par granulométrie laser (BI90 PLUS de BROOKHAVEN (méthode QLS)), de 112 nm, 0,8 % de lécithine de soja et 0,8 % en poids de polymère hydrodispersible selon l'invention (AQ®38S). La suspension, conservée à une température de 45 °C est parfaitement stable pendant au moins 2 mois, alors que la même suspension exempte de polymère hydrodispersible est le siège d'une cristallisation massive en moins de 7 jours de conservation à 45 °C.

## Revendications

1. Suspension colloïdale stable constituée essentiellement :
- d'une phase aqueuse continue,
- de nanosphères de principe actif lipophile ayant une taille moyenne de particules allant de 0,01 à 1 µm,
- d'au moins un agent tensioactif, et
- de particules colloïdales ayant une taille moyenne comprise entre 10 et 500 nm, d'au moins un polymère hydrodispersible.

2. Suspension colloïdale stable selon la revendication 1, **caractérisée par le fait que** le principe actif lipophile est choisi parmi les stérols d'origine végétale et animale et les esters et/ou dérivés partiellement hydrogénés de ceux-ci, la déhydroépiandrostérone (DHEA) et ses précurseurs et dérivés chimiques et biologiques, les acides triterpène pentacycliques, les hydroxystilbènes, les isoflavonoïdes, et les dérivés d'aminophénol de formule 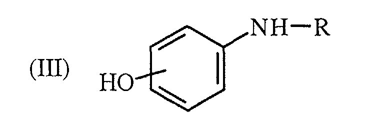 dans laquelle R est un radical correspondant à l'une des formules (i), (ii)
ou (iii) suivantes
(i) -CO-NR₁R₂
(ii) -CO-O-R₃
(iii) -SO₂-R₃
où
R₁ représente un atome d'hydrogène ou un radical alkyle en C₁₋₆, linéaire ou ramifié, saturé ou insaturé, éventuellement hydroxylé,
R₂ représente un atome d'hydrogène ou un radical choisi parmi les radicaux alkyle, saturés ou insaturés, linéaires, cycliques ou ramifiés, en C₁₂ à C₃₀, éventuellement hydroxylés,
R₃ représente un radical choisi parmi les radicaux alkyle en C₁₂ à C₃₀, saturés ou insaturés, linéaires, ramifiés ou cycliques, y compris polycycliques condensés, et éventuellement hydroxylés.

3. Suspension colloïdale stable selon la revendication 2, **caractérisée par le fait que** le principe actif est choisi parmi la déhydroépiandrostérone (DHEA), le sulfate de DHEA, la 7α-hydroxy-DHEA, la 7-céto-DHEA, la prednisolone, la prednisone, la progestérone, la prégnénolone, la testostérone, la diosgénine, l'hécogénine, l'acide ursolique, l'acide oléanolique, le resvératrol (= 3,5,4'-trihydroxystilbène) et le N-cholestéryloxycarbonyl-4-aminophénol, les isoflavonoïdes dont la solubilité dans l'eau à température ambiante (25 °C) est inférieure à 0,01.

4. Suspension colloïdale stable selon l'une quelconque des revendications précédentes, **caractérisée par le fait que** le ou les agents tensioactifs sont choisis parmi les agents tensioactifs non ioniques, anioniques, cationiques, zwitterioniques et leurs mélanges.

5. Suspension colloïdale stable selon l'une quelconque des revendications précédentes, **caractérisée par le fait que** le ou les agents tensioactifs sont choisis parmi
(a) les phospholipides naturels ou de synthèse, hydrogénés ou non, enrichis ou non en phosphatidylcholine,
(b) les stérols polyéthoxylés,
(c) les agents tensio-actifs choisis parmi les esters gras de glycérol, les esters gras de sorbitane, les esters gras de sorbitane polyéthoxylés, les alcools gras polyéthoxylés et les acides gras polyéthoxylés, les chaînes grasses de ces molécules étant saturées, linéaires ou ramifiées ;
(d) le poly(alcool vinylique), la polyvinylpyrrolidone et leurs copolymères,
(e) les polysiloxanes polyéthoxylés, éventuellement polypropoxylés (dénomination CTFA : diméthicone copolyols),
(f) les copolymères diséquencés d'oxyde d'éthylène et d'oxyde de propylène,
(g) les copolymères diséquencés de styrène et d'oxyde d'éthylène et les dérivés sulfates anioniques de ceux-ci,
(h) les esters d'acides gras et de sucres et les éthers d'alcools gras et de sucres et notamment les esters d'acides gras en C₈₋₂₂ et de saccharose, de maltose, de glucose ou de fructose, ou les esters d'acides gras en C₁₄₋₂₂ et de méthylglucose, et leurs mélanges ;
(i) les alcénylsuccinates choisis parmi les alcénylsuccinates polyalcoxylés, les alcénylsuccinates de glucose polyalcoxylés et les alcénylsuccinates de méthylglucose polyalcoxylés,
(j) les acétylènediols polyéthoxylés,
(k) les alkyl(C₁₂₋₃₀)éthersulfates de sodium et les alkyl(C₁₂₋₃₀)sulfates de sodium,
(l) les sels d'ammonium quaternaire ayant des propriétés tensio-actives correspondant à la formule suivante : 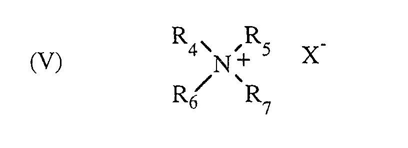
dans laquelle les radicaux R₄, R₅, R₆ et R₇ représentent indépendamment chacun un groupe aliphatique, linéaire ou ramifié ou un groupe aromatique tel qu'un noyau aryle ou alkylaryle, comportant de 1 à 30 atomes de carbone, et X est choisi parmi les anions halogénures, phosphates, acétates, lactates, alkyl(C₂₋₆)-sulfates, alkyl(C₂₋₆)- ou alkyl(C₂₋₆)-arylsulfonates.

6. Suspension colloïdale stable selon l'une quelconque des revendications précédentes, **caractérisée par le fait que** le ou les agents tensioactifs sont présents en une concentration allant de 0,02 à 25 % par rapport au poids total de la suspension.

7. Suspension colloïdale stable selon l'une quelconque des revendications précédentes, **caractérisée par le fait que** les polymères hydrodispersibles sont des polymères synthétiques ou d'origine naturelle, portant des charges anioniques.

8. Suspension colloïdale stable selon la revendication 7, **caractérisée par le fait que** les polymères hydrodispersibles d'origine naturelle sont choisis parmi les dérivés anioniques de la cellulose tels que les esters et éthers anioniques de la cellulose, la résine shellac, la gomme de sandaraque et les dammars.

9. Suspension colloïdale stable selon la revendication 7, **caractérisée par le fait que** les polymères hydrodispersibles synthétiques sont choisis parmi les polyesters, poly(ester amide), polyuréthannes et copolymères vinyliques, portant des fonctions acide carboxylique et/ou acide sulfonique.

10. Suspension colloïdale stable selon la revendication 9, **caractérisée par le fait que** les polymères hydrodispersibles sont choisis parmi les polyesters aromatiques, cycloaliphatiques et/ou aliphatiques portant des fonctions acide sulfonique.

11. Suspension colloïdale stable selon la revendication 10, **caractérisée par le fait que** les polymères hydrodispersibles sont choisis parmi les copolyesters constitués de motifs dérivés d'acide isophtalique, d'acide sulfoisophtalique, de diéthylèneglycol et de 1,4-di(hydroxyméthyl)-cyclohexane.

12. Suspension colloïdale stable selon la revendication 10, **caractérisée par le fait que** les polymères hydrodispersibles sont choisis parmi les copolyesters constitués de motifs dérivés d'acide isophtalique, d'acide sulfoisophtalique, d'éthylèneglycol et d'acide téréphtalique.

13. Suspension colloïdale stable selon l'une des revendications 10 à 12, **caractérisée par le fait que** la fraction de motifs dérivés d'acide sulfoisophtalique dans le polymère va de 2 à 20 % en poids.

14. Suspension colloïdale stable selon l'une quelconque des revendications précédentes, **caractérisée par le fait que** la taille moyenne des particules colloïdales de polymère hydrodispersible va de 20 à 400 nm.

15. Suspension colloïdale stable selon l'une quelconque des revendications précédentes, **caractérisée par le fait que** le rapport en poids polymère hydrodispersible/nanosphères de principe actif lipophile va de 1/100 à 1/1 et de préférence de 1/50 à 1/2.

16. Composition à application topique contenant, dans un milieu physiologiquement acceptable, une suspension colloïdale stable selon l'une quelconque des revendications précedentes.

17. Composition à application topique selon la revendication 16, **caractérisée par le fait qu'**elle contient de 0,1 à 40 % en poids, de préférence de 1 à 30 % en poids de suspension colloïdale stable selon l'une quelconque des revendications 1 à 14.

18. Utilisation de polymères hydrodispersibles sous forme de particules colloïdales ayant une taille moyenne allant de 10 à 500 nm pour stabiliser des suspensions aqueuses de nanosphères de principe actif lipophile, ayant une taille moyenne de particules allant de 0,01 à 1 µm, contre la recristallisation du principe actif.

19. Utilisation selon la revendication 18, **caractérisée par le fait que** le principe actif lipophile est choisi parmi les stérols d'origine végétale et animale et les esters et/ou dérivés partiellement hydrogénés de ceux-ci, la déhydroépiandrostérone (DHEA) et ses précurseurs et dérivés chimiques et biologiques, les acides triterpène pentacycliques, les hydroxystilbènes, les isoflavonoïdes, et les dérivés d'aminophénol de formule 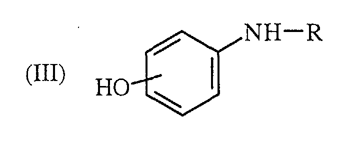 dans laquelle R est un radical correspondant à l'une des formules (i), (ii)
ou (iii) suivantes
(i) -CO-NR₁R₂
(ii) -CO-O-R₃
(iii) -SO₂-R₃
où
R₁ représente un atome d'hydrogène ou un radical alkyle en C₁₋₆, linéaire
ou ramifié, saturé ou insaturé, éventuellement hydroxylé,
R₂ représente un atome d'hydrogène ou un radical choisi parmi les radicaux alkyle, saturés ou insaturés, linéaires, cycliques ou ramifiés, en C₁₂ à C₃₀, éventuellement hydroxylés,
R₃ représente un radical choisi parmi les radicaux alkyle en C₁₂ à C₃₀, saturés ou insaturés, linéaires, ramifiés ou cycliques, y compris polycycliques condensés, et éventuellement hydroxylés.

20. Utilisation selon la revendication 19, **caractérisée par le fait que** le principe actif est choisi parmi la déhydroépiandrostérone (DHEA), le sulfate de DHEA, la 7α-hydroxy-DHEA, la 7-céto-DHEA, la prednisolone, la prednisone, la progestérone, la prégnénolone, la testostérone, la diosgénine, l'hécogénine, l'acide ursolique, l'acide oléanolique, le resvératrol (= 3,5,4'-trihydroxystilbène) et le N-cholestéryloxycarbonyl-4-aminophénol, les isoflavonoïdes dont. la solubilité dans l'eau à température ambiante (25 °C) est inférieure à 0,01.

21. Utilisation selon l'une quelconque des revendications 18 à 20, **caractérisée par le fait que** les polymères hydrodispersibles sont des polymères synthétiques ou d'origine naturelle, portant des charges anioniques.

22. Utilisation selon la revendication 21, **caractérisée par le fait que** les polymères hydrodispersibles d'origine naturelle sont choisis parmi les dérivés anioniques de la cellulose tels que les esters et éthers anioniques de la cellulose, la résine shellac, la gomme de sandaraque et les dammars.

23. Utilisation selon la revendication 21, **caractérisée par le fait que** les polymères hydrodispersibles synthétiques sont choisis parmi les polyesters, poly(ester amide), polyuréthannes et copolymères vinyliques, portant des fonctions acide carboxylique et/ou acide sulfonique.

24. Utilisation selon la revendication 23, **caractérisée par le fait que** les polymères hydrodispersibles sont choisis parmi les polyesters aromatiques, cycloaliphatiques et/ou aliphatiques portant des fonctions acide sulfonique.

25. Utilisation selon la revendication 24, **caractérisée par le fait que** les polymères hydrodispersibles sont choisis parmi les copolyesters constitués de motifs dérivés d'acide isophtalique, d'acide sulfoisophtalique, de diéthylèneglycol et de 1,4-di(hydroxyméthyl)-cyclohexane.

26. Utilisation selon la revendication 25, **caractérisée par le fait que** les polymères hydrodispersibles sont choisis parmi les copolyesters constitués de motifs dérivés d'acide isophtalique, d'acide sulfoisophtalique, d'éthylèneglycol et d'acide téréphtalique.

27. Utilisation selon l'une des revendications 24 à 26, **caractérisée par le fait que** la fraction de motifs dérivés d'acide sulfoisophtalique dans le polymère va de 2 à 20 % en poids.

28. Utilisation selon l'une quelconque des revendications 18 à 27, **caractérisée par le fait que** la taille moyenne des particules colloïdales de polymère hydrodispersible va de 20 à 400 nm.

29. Utilisation selon l'une quelconque des revendications 18 à 28, **caractérisée par le fait que** le rapport en poids polymère hydrodispersible/nanosphères de principe actif lipophile va de 1/100 à 1/1 et de préférence de 1/50 à 1/2.

30. Utilisation cosmétique de la composition selon l'une des revendications 16 et 17, pour le traitement, le soin et/ou le maquillage de la peau du visage et/ou du corps, des muqueuses, du cuir chevelu et/ou des fibres kératiniques.

31. Procédé de traitement cosmétique des matières kératiniques humaines comme la peau, y compris le cuir chevelu, les cheveux, les cils, les sourcils, les ongles ou les muqueuses, notamment les lèvres, **caractérisé par le fait qu'**on applique sur les matières kératiniques une composition selon l'une des revendications 16 et 17.
